# DEMANDE DE BREVET EUROPEEN

(11) **EP 2 105 121 A1**
(43) Date de publication de la demande: **30.09.2009**
(21) Numéro de dépôt: 09155501.1
(22) Date de dépôt: 18.03.2009
(51) Int. Cl.: A61K 8/04, A61K 8/22, A61K 8/37, A61K 8/39, A61K 8/42, A61Q 5/04, A61Q 5/08, A61Q 5/10

(54) **Dispersion aqueuse oxydante pour le traitement des fibres keratiniques comprenant un compose amphiphile non ionique a caractere hydrophobe**

(30) Priorité: 28.03.2008 FR 0852036
(71) Demandeur: L'Oréal, 75008 Paris (FR)
(72) Inventeur: Simonet, Frédéric, 92110, Clichy (FR); Nicolas-Morgantini, Luc, 60810, Rully (FR)
(74) Mandataire: Prevel, Estelle Nicole

(57) **Abrégé**

La présente invention a pour objet une composition pour le traitement des fibres kératiniques sous forme de dispersion aqueuse comprenant, dans un milieu cosmétiquement acceptable, un ou plusieurs agents oxydants ; un ou plusieurs composés amphiphiles non ioniques à caractère hydrophobe solides à température ambiante ; et éventuellement un ou plusieurs agents tensioactifs à caractère hydrophile ; le rapport pondéral entre les agents tensioactifs à caractère hydrophile et les composés amphiphiles non ioniques à caractère hydrophobe étant compris entre 0 et 0,16.

Outre sa simplicité, la composition conforme à l'invention est stable en conservation. En particulier, on n'observe pas de variations notables de l'aspect microscopique ou des propriétés rhéologiques au cours du temps. Elle peut se présenter sous forme de crème d'aspect particulièrement brillant et très facile à étaler sur la chevelure ou à mélanger avec d'autres milieux (crèmes de coloration d'oxydation, pâtes décolorantes...).

Ainsi, les compositions de teinture, de décoloration ou de déformation permanente obtenues à partir de cette composition sont stables et présentent des qualités d'application et d'usage améliorées et plus performantes.

## Description

La présente invention a pour objet une composition pour le traitement des fibres kératiniques, et en particulier des fibres kératiniques humaines telles que les cheveux, sous forme de dispersion aqueuse comprenant, dans un milieu cosmétiquement acceptable, un ou plusieurs agents oxydants ; un ou plusieurs composés amphiphiles non ioniques à caractère hydrophobe solides à température ambiante ; et éventuellement un ou plusieurs agents tensioactifs à caractère hydrophile ; le rapport pondéral entre les agents tensioactifs à caractère hydrophile et les composés amphiphiles non ioniques à caractère hydrophobe étant compris entre 0 et 0,16.

En cosmétique, dans les domaines de la teinture, de la décoloration et de la déformation permanente des fibres kératiniques, et en particulier des fibres kératiniques humaines telles que les cheveux, on utilise des compositions oxydantes.

Ainsi, en teinture d'oxydation des cheveux, des compositions oxydantes sont mélangées aux colorants d'oxydation (bases et coupleurs), qui sont incolores par eux-mêmes, pour engendrer des composés colorés et colorants par un processus de condensation oxydative. Des compositions oxydantes sont également utilisées en teinture directe des cheveux en mélange avec certains colorants directs qui sont colorés et colorants pour obtenir une coloration avec un effet éclaircissant des cheveux. Parmi les agents oxydants classiquement utilisés pour la teinture des fibres kératiniques, on peut citer le peroxyde d'hydrogène ou des composés susceptibles de produire le peroxyde d'hydrogène par hydrolyse tels que le peroxyde d'urée, les persels comme les perborates et persulfates, le peroxyde d'hydrogène étant plus particulièrement préféré.

En décoloration des cheveux, les compositions de décoloration contiennent un ou plusieurs agents oxydants. Parmi ces agents oxydants, les plus classiquement utilisés sont le peroxyde d'hydrogène ou des composés susceptibles de produire le peroxyde d'hydrogène par hydrolyse, tels que le peroxyde d'urée ou les persels comme les perborates, les percarbonates et les persulfates, le peroxyde d'hydrogène et les persulfates étant particulièrement préférés.

Ces compositions peuvent être des compositions aqueuses contenant des agents alcalins (amines ou ammoniaque) que l'on dilue au moment de l'emploi avec une composition aqueuse de peroxyde d'hydrogène.

Ces compositions peuvent aussi être formées de produits anhydres qui contiennent des composés alcalins (amines et silicates alcalins), et un réactif peroxygéné tels que les persulfates, perborates ou percarbonates, d'ammonium ou de métaux alcalins, que l'on dilue au moment de l'emploi avec une composition aqueuse de peroxyde d'hydrogène.

En déformation permanente des cheveux, dans un premier temps, on réalise l'ouverture des liaisons disulfures -S-S- de la kératine (cystine) à l'aide d'une composition contenant un agent réducteur adapté (étape de réduction) puis, après avoir rincé la chevelure ainsi traitée, on reconstitue dans un second temps les liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou des shampooings, et ceci par opposition aux simples techniques classiques de déformation temporaire, telles que de mise en pli.

Les compositions oxydantes nécessaires à la mise en oeuvre de l'étape de fixation, sont le plus souvent des compositions à base d'eau oxygénée.

La plupart des supports actuels utilisés pour les compositions oxydantes sont formulés à partir de composés cristallisables, comme par exemple l'alcool cétylstéarylique. Ces composés sont généralement fondus, introduits en phase aqueuse et émulsionnés à chaud. L'émulsion est ensuite refroidie, provoquant la cristallisation de ces composés sous forme de particules solides de taille micrométrique (cristaux). De manière à réaliser l'étape d'émulsification, des tensioactifs comme par exemple des tensioactifs non ioniques fortement oxyéthylénés (environ 20 à 30 unités oxyéthylène) sont systématiquement introduits dans les formules. Ils ont pour rôle d'affiner l'émulsion et d'aboutir en final à une dispersion lisse et onctueuse, exempte de grumeaux.

Il s'avère que de manière générale, ce type de support évolue lors des conservations. On note alors une évolution de l'aspect microscopique, avec l'apparition de nouvelles morphologies de cristaux, plus massives. Les propriétés rhéologiques telles que la viscosité, la contrainte seuil ou les modules viscoélastiques peuvent également évoluer, à la hausse comme à la baisse.

Le but de la présente invention est de fournir de nouvelles compositions oxydantes qui ne présentent pas les inconvénients décrits ci-dessus, notamment des compositions oxydantes qui sont stables dans le temps.

Ce but est atteint avec la présente invention qui a pour objet une composition pour le traitement des fibres kératiniques sous forme de dispersion aqueuse comprenant, dans un milieu cosmétiquement acceptable :
- un ou plusieurs agents oxydants ;
- un ou plusieurs composés amphiphiles non ioniques à caractère hydrophobe solides à température ambiante ; et
- éventuellement un ou plusieurs agents tensioactifs à caractère hydrophile ;
   le ou les agents tensioactifs à caractère hydrophile présents dans la composition étant cationiques ou zwittérioniques ;
   le rapport pondéral entre les agents tensioactifs à caractère hydrophile et les composés amphiphiles non ioniques à caractère hydrophobe étant compris entre 0 et 0,16.

Le ou les composés amphiphiles non ioniques à caractère hydrophobe peuvent facilement s'émulsionner à chaud dans l'eau, à la manière des mélanges alcool gras / tensioactif non ionique fortement oxyéthyléné. Leur intérêt résulte dans le fait qu'un état d'équilibre est atteint dès la fin de fabrication, aucune réorganisation n'a lieu entre les différentes molécules qui constituent les cristaux.

Par conséquent, outre sa simplicité, la composition conforme à l'invention est plus stable en conservation. En particulier, on n'observe pas de variations notables de l'aspect microscopique ou des propriétés rhéologiques au cours du temps. Elle peut se présenter sous forme de crème d'aspect particulièrement brillant et très facile à étaler sur la chevelure ou à mélanger avec d'autres milieux (crèmes de coloration d'oxydation, pâtes décolorantes...).

Ainsi, les compositions de teinture, de décoloration ou de déformation permanente obtenues à partir de la composition oxydante conforme à l'invention sont stables et présentent des qualités d'application et d'usage améliorées et plus performantes.

Lorsque la composition conforme à l'invention est utilisée pour la coloration des fibres kératiniques, on obtient de bonnes propriétés tinctoriales, notamment des colorations puissantes, chromatiques, peu sélectives et résistant bien aux diverses agressions que peuvent subir les cheveux telles que les shampooings, la lumière, la sueur et les déformations permanentes.

Lorsque la composition conforme à la présente invention est utilisée pour la décoloration des fibres kératiniques, elle permet d'obtenir un bon effet d'éclaircissement de ces fibres sans les dégrader et sans altérer leurs propriétés cosmétiques.

Lorsque la composition conforme à la présente invention est utilisée pour la déformation permanente des fibres kératiniques, elle permet d'obtenir une déformation permanente satisfaisante de ces fibres sans les dégrader et sans altérer leurs propriétés cosmétiques.

L'invention a aussi pour objet un procédé de traitement des fibres kératiniques, notament un procédé de coloration, de décoloration ou de déformation permanente des fibres kératiniques, mettant en oeuvre cette composition oxydante.

Un autre objet de l'invention est l'utilisation de cette composition oxydante pour le traitement des fibres kératiniques, notamment la coloration, la décoloration ou la déformation permanente des fibres kératiniques.

Dans ce qui suit, sauf indication contraire, les bornes des intervalles indiqués sont comprises dans l'invention.

Dans le sens de la présente invention, on entend par « dispersion » une suspension de particules solides dans un liquide.

La composition conforme à l'invention s'émulsionne aisément, par exemple à chaud à l'aide de moyens d'agitation traditionnels comme des turbines rotor / stator, puis elle est refroidie à température ambiante de manière à former les particules solides.

Au sens de la présente invention, on entend par « température ambiante » une température de 25 °C plus ou moins 5 °C.

Le ou les agents oxydants présents dans la composition conforme à l'invention sont choisis parmi le peroxyde d'hydrogène, le peroxyde d'urée, les bromates alcalins, les polythionates, les persels, tels que les perborates, les percarbonates et les persulfates.

De préférence, l'agent oxydant est le peroxyde d'hydrogène ou un bromate.

Le ou les agents oxydants représentent généralement de 0,1 à 50 %, de préférence de 1 à 20 % en poids par rapport au poids total de la composition oxydante.

Selon un mode de réalisation particulier de l'invention, lorsque l'agent oxydant est le peroxyde d'hydrogène, la composition oxydante comprend un ou plusieurs agents stabilisants de l'eau oxygénée.

A titre d'exemples d'agents stabilisants de l'eau oxygénée, on peut citer en particulier les pyrophosphates des métaux alcalins ou alcalino-terreux tels que le pyrophosphate de tétrasodium, les stannates des métaux alcalins ou alcalino-terreux, la phénacétine ou les sels d'acides et d'oxyquinoléine comme le sulfate d'oxyquinoléine. De préférence, on utilise un ou plusieurs stannates en association ou non avec un ou plusieurs pyrophosphates.

Le ou les agents stabilisants de l'eau oxygénée représentent généralement de 0,0001 à 5 % en poids et de préférence de 0,01 à 2 % en poids par rapport au poids total de la composition oxydante.

Dans le cadre de la présente invention, on entend par « composés amphiphiles » des composés hydrodispersibles présentant une ou plusieurs parties polaires et une ou plusieurs parties apolaires.

Selon un mode de réalisation particulier de l'invention, le ou les composés amphiphiles non ioniques à caractère hydrophobe comportent une ou plusieurs chaînes grasses saturées en C₁₂-C₃₀ reliées par une liaison éther, ester, amide ou carbamate à une tête polaire composée d'une ou plusieurs unités choisies parmi les unités oxyalkylène, sorbitane, sucre et autres polyols.

A titre d'exemples d'unités oxyalkylène, on peut citer les unités oxyéthylène et oxypropylène.

A titre d'exemples d'unités sucre, on peut citer les unités glucose et fructose.

A titre d'exemples d'autres unités polyol, on peut citer les unités glycérol, tétritol, pentitol, sorbitol, mannitol et hexitol.

Par « composé amphiphile non ionique à caractère hydrophobe », on entend au sens de la présente invention tout composé amphiphile non ionique qui à température ambiante présente une solubilité dans l'eau inférieure à 2 %, et de préférence inférieure à 1 %.

Selon un mode de réalisation particulier de l'invention, le ou les composés amphiphiles non ioniques à carractère hydrophobe ont une HLB calculée comprise entre 2 et 10. La HLB calculée se définit comme étant le coefficient suivant :
HLB calculée = 20 x masse molaire de la partie hydrophile / masse molaire totale

Pour un alcool gras oxyéthyléné, la partie hydrophile correspond aux unités oxyéthylène condensées sur l'alcool gras et la HLB calculée correspond alors à la HLB selon Griffin (Griffin W. C., J. Soc. Cosmet. Chemists, 5, 249, 1954). Pour un ester ou un amide, la partie hydrophile est naturellement définie comme se trouvant au-delà du groupement carbonyle, en partant de la ou des chaîne(s) grasse(s). Bien entendu, cette grandeur est additive et pour un mélange de molécules, la HLB calculée correspond à la moyenne pondérée en masse des HLB calculées pour chaque molécule.

Le ou les composés amphiphiles non ioniques à caractère hydrophobe ont un point de fusion suffisamment élevé pour être aptes à former dans l'eau des particules solides à température ambiante.

Selon un mode de réalisation particulier de l'invention, le point de fusion du ou des composés amphiphiles non ioniques à caractère hydrophobe est supérieur à 30 °C, et plus préférentiellement, supérieur à 40 °C.

A titre d'exemples de composés non ioniques à caractère hydrophobe pouvant être utilisés dans la présente invention, on peut citer les composés suivants :

| Nom INCl | Référence commerciale | HLB calculée |
|---|---|---|
| STEARETH-2 | Brij 72 (Uniqema) | 4,9^{*} |
| STEARETH-3 | Isoxal 5 (Vevy) | 6,6^{*} |
| STEARETH-4 | Nikkol BS-4 (Nikko) | 7,9^{*} |
| STEARETH-5 | Jeecol SA-5 (Jeen) | 9,0^{*} |
| STEARETH-6 | Emalex 606 (Nihon Emulsion) | 9,9^{*} |
| CETETH-2 | Brij 52 (Uniqema) | 5,3^{*} |
| CETETH-3 | Emalex 103 (Nihon Emulsion) | 7,1^{*} |
| CETETH-4 | Lipocol C-4 (Lipo) | 8,4^{*} |
| CETETH-5 | Volpo C5 (Croda) | 9,5^{*} |
| CETEARETH-2 | Volpo CS2 (Croda) | 5,1^{*} |
| CETEARETH-3 | Jeecol CS-3 (Jeen) | 6,8^{*} |
| CETEARETH-4 | Lipocol SC-4 (Lipo) | 8,1^{*} |
| CETEARETH-5 | Volpo CS5 (Croda) | 9,2^{*} |
| BEHENETH-5 | Nikkol BB-5 (Nikko) | 8,1^{*} |
| COCAMIDE MEA | Comperlan 100 (Cognis) | 4,8 |
| COCAMIDE MIPA | Ninol M-10 (Stepan) | 5,6 |
| COCAMIDE DEA | Comperlan KD (Cognis) | 7,1 |
| STEARAMIDE MEA | Monamid S (Uniqema) | 3,7 |
| STEARAMIDE DEA | Lipamide S (Lipo) | 5,6 |
| MYRISTAMIDE DEA | Jeemide MRCA (Jeen) | 6,6 |
| MYRISTAMIDE MEA | Witcamide MM (Witco) | 4,4 |
| POLYGLYCERYL-2 DISTEARATE | Emalex DSG-2 (Ikeda) | 4,7 |
| POLYGLYCERYL-3 DISTEARATE | Cithrol 2623 (Croda) | 6,2 |
| POLYGLYCERYL-2 STEARATE | Nikkol DGMS (Nikko) | 7,6 |
| POLYGLYCERYL-3 STEARATE | Radiasurf 7248 (Atofina) | 9,4 |
| PEG-2 STEARATE | Sedefos 75 (Gattefosse) | 5,6 |
| PEG-3 STEARATE | Tegin D 1102 (Goldschmidt) | 7,2 |
| PEG-4 STEARATE | Cithrol 2MS (Croda) | 8,4 |
| SORBITAN DISTEARATE | Sorbon S-66 (Toho) | 4,7 |
| SORBITAN PALMITATE | Span 40 (Uniqema) | 8,1 |
| SORBITAN STEARATE | Span 60 (Uniqema) | 7,6 |
| SORBITAN TRISTEARATE | Span 65 (Uniqema) | 3,3 |
| MYRISTYL GLUCOSIDE | Montanov 14 (Seppic) | 9,5 |
| CETEARYL GLUCOSIDE | Tego Care CG 90 (Degussa) | 8,6 |
| ARACHIDYL GLUCOSIDE | Montanov 202 (Seppic) | 7,8 |

| | | |
|---|---|---|
| ^{*} correspondant à la HLB calculée selon la méthode de Griffin | | |

Le ou les composés amphiphiles non ioniques à caractère hydrophobe représentent généralement de 2 à 30 %, de préférence de 2 à 20 %, et encore plus préférentiellement de 2 à 10 % en poids par rapport au poids total de la composition oxydante.

Par « agent tensioactif à caractère hydrophile », on entend au sens de la présente invention des agents tensioactifs ayant une HLB calculée supérieure à 10, de préférence comprise entre 10 et 50.

Le ou les agents tensioactifs à caractère hydrophile utiles dans le cadre de l'invention sont cationiques ou zwittérioniques.

Le ou les agents tensioactifs à caractère hydrophile cationiques peuvent être choisis par exemple parmi les sels de monoalkyl(C₈-C₃₀) triméthyl ammonium, les diesters quaternaires.

Le ou les agents tensioactifs à caractère hydrophile zwittérioniques peuvent être en particulier choisis parmi les alkyl bétaïnes, les alkyl amido alkyl bétaïnes, les sultaïnes, les phospho bétaïnes, les amphodiacétates, de préférence les alkyl bétaïnes, les amphodiacétates.

Dans la composition conforme à l'invention, le rapport pondéral entre les agents tensioactifs à caractère hydrophile et les composés amphiphiles non ioniques à caractère hydrophobe est compris entre 0 et 0,16. De préférence, ce rapport pondéral est compris entre 0 et 0,10, et encore plus préférentiellement entre 0 et 0,05.

Selon un mode de réalisation préféré de l'invention, la composition ne comprend pas d'agent tensioactif à caractère hydrophile.

Selon un mode de réalisation particulier de l'invention, la composition comprend un ou plusieurs alcools gras en C₁₂-C₃₀ de HLB calculée inférieure à 2.

Plus particulièrement, le ou les alcools gras sont choisis parmi les alcools non (poly)oxyalkylénés et non (poly)glycérolés, comprenant une ou plusieurs chaînes grasses présentant de 12 à 30 atomes de carbone, plus particulièrement de 14 à 22 atomes de carbone, et de façon encore plus avantageuse, de 16 à 18 atomes de carbone, linéaires ou ramifiés, saturés ou insaturés, les chaînes grasses étant éventuellement substituées par un ou deux groupements hydroxyle supplémentaires. Lorsque l'alcool est insaturé, il comprend de 1 à 3 doubles liaisons carbone-carbone (-C=C-), conjuguées ou non. De préférence, l'alcool gras est un monoalcool saturé.

A titre d'exemples d'alcools gras, on peut citer l'alcool laurique, cétylique, stéarylique, béhénique, myristique, linoléïque, undécylénique, palmitoléïque, linolénique, arachidonique, érucique, isocétylique, isostéarylique, isobéhénylique, l'alcool oléique et leurs mélanges.

De préférence, le ou les alcools gras sont choisis parmi l'alcool stéarylique, l'alcool béhénique, l'alcool cétylique.

Le ou les alcools gras en C₁₂-C₃₀ représentent généralement de 0 à 10 %, de préférence de 0 à 2 %, et encore plus préférentiellement de 0 à 0,5 % en poids par rapport au poids total de la composition oxydante.

Selon un mode de réalisation préféré de l'invention, la composition ne comprend pas d'alcool gras en C₁₂-C₃₀.

Par milieu cosmétiquement acceptable, on entend, au sens de la présente invention, un milieu compatible avec les fibres kératiniques, et en particulier les fibres kératiniques humaines telles que les cheveux.

Le milieu cosmétiquement acceptable de la composition conforme à la présente invention comprend généralement de l'eau ou un mélange d'eau et d'un ou plusieurs solvants organiques. A titre de solvants organiques, on peut par exemple citer les alcanols inférieurs en C₁-C₄, tels que l'éthanol et l'isopropanol ; les alcools aromatiques comme l'alcool benzylique ou le phénoxyéthanol ; les polyols ou éthers de polyols tels que les éthers monométhylique, monoéthylique et monobutylique d'éthylèneglycol, le propylèneglycol ou ses éthers tels que le monométhyléther de propylèneglycol, le butylèneglycol, le dipropylèneglycol ainsi que les alkyléthers de diéthylèneglycol comme le monoéthyléther ou le monobutyléther du diéthylèneglycol, ou encore le glycérol ; ainsi que leurs mélanges.

Les solvants sont, de préférence, présents dans des proportions comprises entre 0,1 et 35 % en poids environ par rapport au poids total de la composition oxydante, et encore plus préférentiellement entre 1 et 40 % en poids environ.

La composition conforme à l'invention peut également comprendre des composés additionnels utilisés classiquement en cosmétique. Ces composés peuvent notamment être choisis parmi les polymères épaississants ou stabilisants, les polymères conditionneurs non siliconés, les silicones, les chélatants, ainsi que les parfums.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

La composition selon l'invention peut se présenter sous des formes diverses, telles que sous forme de crème, de gel, de lait, de lotion, de mousse, ou sous toute autre forme appropriée pour réaliser le traitement des fibres kératiniques, et notamment des fibres kératiniques humaines telles que les cheveux. De préférence, elle se présente sous la forme d'une crème ou d'un lait.

Le pH de la composition oxydante selon l'invention varie généralement de 1,5 à 4,5, et de préférence de 2 à 3,5. Il peut être ajusté par ajout d'agents acidifiants tels que l'acide chlorhydrique, l'acide acétique, l'acide lactique, l'acide borique, l'acide citrique et l'acide phosphorique ou d'agents acidifiants en présence d'agents alcalins.

Un autre objet de l'invention est un procédé de traitement des fibres kératiniques comprenant l'application sur les fibres kératiniques d'une composition oxydante telle que définie précédemment.

La composition oxydante conforme à l'invention peut par exemple être utilisée dans un procédé de teinture des fibres kératiniques, et en particulier des fibres kératiniques humaines telles que les cheveux.

Le procédé de teinture des fibres kératiniques conforme à l'invention met en oeuvre une composition colorante comprenant dans un support approprié pour la teinture des fibres kératiniques un ou plusieurs colorants directs et / ou un ou plusieurs colorants d'oxydation et une composition oxydante telle que définie ci-dessus.

Selon ce procédé, on applique sur les fibres kératiniques la composition colorante, la couleur étant révélée à pH acide, neutre ou alcalin à l'aide d'une composition oxydante selon l'invention qui est appliquée simultanément ou séquentiellement, avec ou sans rinçage intermédiaire.

Selon une forme de mise en oeuvre particulièrement préférée du procédé de teinture selon l'invention, on mélange, au moment de l'emploi, la composition colorante avec une composition oxydante selon l'invention. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

Le ou les colorants directs peuvent être choisis parmi les colorants directs classiquement utilisés en coloration directe. A titre d'exemples, ces colorants directs sont choisis parmi les colorants nitrés de la série benzénique, les colorants directs azoïques, les colorants directs méthiniques, les colorants directs quinoniques, les colorants directs aziniques, les colorants directs triarylméthaniques, les colorants directs indoaminiques, les colorants directs naturels. Ces colorants directs peuvent être de nature non ionique, anionique ou cationique.

Parmi les colorants directs benzéniques, on peut citer le 1,4-diamino-2-nitrobenzène, le 1-amino-2-nitro-4-(β-hydroxyéthylamino)-benzène, le 1-amino-2-nitro-4-bis(β-hydroxyéthyl)-aminobenzène, le 1,4-bis(β-hydroxyéthylamino)-2-nitro-benzène, le 1-β-hydroxyéthytamino-2-nitro-4-bis-(β-hydroxyéthytamino)-benzène, le 1-β-hydroxyéthylamino-2-nitro-4-amino-benzène, le 1-β-hydroxyéthytamino-2-nitro-4-(éthyt)(β-hydroxyéthyl)-aminobenzène, le 1-amino-3-méthyl-4-β-hydroxyéthylamino-6-nitro-benzène, le 1-amino-2-nitro-4-β-hydroxyéthylamino-5-chloro-benzène, le 1,2-diamino-4-nitro-benzène, le 1-amino-2-β-hydroxyéthylamino-5-nitro-benzène, le 1,2-bis-(β-hydroxyéthylamino)-4-nitro-benzène, le 1-amino-2-[tris-(hydroxyméthyl)-méthylamino]-5-nitro-benzène, le 1-hydroxy-2-amino-5-nitro-benzène, le 1-hydroxy-2-amino-4-nitro-benzène, le 1-hydroxy-3-nitro-4-amino-benzène, le 1-hydroxy-2-amino-4,6-dinitro-benzène, le 1-β-hydroxyéthyloxy-2-β-hydroxyéthylamino-5-nitro-benzène le 1-méthoxy-2-β-hydroxyéthylamino-5-nitro-benzène, le 1-β-hydroxyéthyloxy-3-méthylamino-4-nitrobenzène, le 1-β,γ-dihydroxypropyloxy-3-méthylamino-4-nitrobenzène, le 1-β-hydroxyéthylamino-4-β,γ-dihydroxypropyloxy-2-nitro-benzène, le 1-β,γ-dihydroxypropylamino-4-trifluorométhyl-2-nitro-benzène, le 1-β-hydroxyéthylamino-4-trifluorométhyl-2-nitro-benzène, le 1-β-hydroxyéthylamino-3-méthyl-2-nitro-benzène, le 1-β-aminoéthylamino-5-méthoxy-2-nitro-benzène, le 1-hydroxy-2-chloro-6-éthylamino-4-nitro-benzène, le 1-hydroxy-2-chloro-6-amino-4-nitro-benzène, le 1-hydroxy-6-[bis-(β-hydroxyéthyl)-amino]-3-nitro-benzène, le 1-β-hydroxyéthylamino-2-nitro-benzène, le 1-hydroxy-4-β-hydroxyéthylamino-3-nitro-benzène.

Parmi les colorants directs azoïques, on peut citer les colorants azoïques cationiques décrits dans les demandes de brevets WO 95/15144, WO-95/01772, EP 0 714 954, FR 2 822 696, FR 2 825 702, FR 2 825 625, FR 2 822 698, FR 2 822 693, FR 2 822 694, FR 2 829 926, FR 2 807 650, WO 02/078660, WO 02/100834, WO 02/100369 et FR 2 844 269 dont le contenu fait partie intégrante de l'invention.

Parmi ces composés, on peut tout particulièrement citer le chlorure de 1,3-diméthyl-2-[[4-(diméthylamino)phényl]azo]-1H-Imidazolium, le chlorure de 1,3-diméthyl-2-[(4-aminophényl)azo]-1H-Imidazolium, le méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3^{e} édition : Disperse Red 17, Acid Yellow 9, Acid Black 1, Basic Red 22, Basic Red 76, Basic Yellow 57, Basic Brown 16, Acid Yellow 36, Acid Orange 7, Acid Red 33, Acid Red 35, Basic Brown 17, Acid Yellow 23, Acid Orange 24, Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques, on peut citer les colorants suivants : Disperse Red 15, Solvent Violet 13, Acid Violet 43, Disperse Violet 1, Disperse Violet 4, Disperse Blue 1, Disperse Violet 8, Disperse Blue 3, Disperse Red 11, Acid Blue 62, Disperse Blue 7, Basic Blue 22, Disperse Violet 15, Basic Blue 99, ainsi que les composés suivants : la 1-N-méthylmorpholiniumpropylamino-4-hydroxyanthraquinone, la 1-aminopropylamino-4-méthylaminoanthraquinone, la 1-aminopropylaminoanthraquinone, la 5-β-hydroxyéthyt-1,4-diaminoanthraquinone, la 2-aminoéthylaminoanthraquinone, la 1,4-bis-(β,γ-dihydroxypropylamino)-anthraquinone.

Parmi les colorants aziniques, on peut citer les composés suivants : Basic Blue 17, Basic Red 2.

Parmi les colorants triarylméthaniques, on peut citer les composés suivants : Basic Green 1, Acid blue 9, Basic Violet 3, Basic Violet 14, Basic Blue 7, Acid Violet 49, Basic Blue 26, Acid Blue 7.

Parmi les colorants indoaminiques, on peut citer les composés suivants : la 2-β-hydroxyéthlyamino-5-[bis-(β-4'-hydroxyéthyl)amino]anilino-1,4-benzoquinone, la 2-β-hydroxyéthylamino-5-(2'-méthoxy-4'-amino)anilino-1,4-benzoquinone, la 3-N(2'-Chloro-4'-hydroxy)phényl-acétylamino-6-méthoxy-1,4-benzoquinone imine, la 3-N(3'-Chloro-4'-méthylamino)phényl-uréido-6-méthyl-1,4-benzoquinone imine, la 3-[4'-N-(Ethyl,carbamylméthyl)-amino]-phényl-uréido-6-méthyl-1,4-benzoquinone imine.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine, les orcéines. On peut également utiliser les extraits ou décoctions contenant ces colorants naturels et notamment les cataplasmes ou extraits à base de henné.

Le ou les colorants directs sont généralement présents dans la composition colorante en quantité comprise entre 0,001 et 20 % en poids environ du poids total de la composition, et encore plus préférentiellement entre 0,005 et 10 % en poids environ.

Le ou les colorants d'oxydation peuvent être choisis parmi les bases d'oxydation et les coupleurs conventionnellement utilisés dans le domaine de la coloration.

A titre d'exemples de bases d'oxydation, on peut citer les para-phénylènediamines, les bases doubles, les para-aminophénols, les ortho-aminophénols, les bases hétérocycliques et leurs sels d'addition.

Parmi les para-phénylènediamines, on peut citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylène-diamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bases doubles, on peut citer, à titre d'exemples, les bis-phénylalkylènediamines et les bis-para-aminophénols.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(P-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

A titre de dérivés pyrazoliques, on peut aussi citer les diamino-N,N-dihydropyrazopyrazolones et notamment celles décrites dans la demande FR 2 886 136 telles que les composés suivants et leurs sels d'addition.

Parmi ces composés, les préférés sont les suivants :
2,3-Diamino-6,7-dihydro-1 H,5H-pyrazolo[1,2-a]pyrazol-1-one,
2-Amino-3-éthylamino-6,7-dihydro-1 H,5H-pyrazolo[1,2-a]pyrazol-1-one,
2-Amino-3-isopropylamino-6,7-dihydro-1H,5H-pyrazolo[1,2-a]pyrazol-1-one,
2-Amino-3-(pyrrolidin-1-yl)-6,7-dihydro-1H,5H-pyrazolo[1,2-a]pyrazol-1-one,
4,5-diamino-1,2-diméthyl-1,2-dihydro-pyrazol-3-one,
4,5-diamino-1,2-diéthyl-1,2-dihydro-pyrazol-3-one,
4,5-diamino-1,2-di-(2-hydroxyéthyl)-1,2-dihydro-pyrazol-3-one,
2-amino-3-(2-hydroxyéthyl)amino-6,7-dihydro-1H,5H-pyrazolo[1,2-a]pyrazol-1-one,
2-amino-3-diméthylamino-6,7-dihydro-1H,5H-pyrazolo[1,2-a]pyrazol-1-one,
2,3-diamino-5,6,7,8-tétrahydro-1 H,6H-pyridazino[1,2-a]pyrazol-1-one,
4-Amino-1,2-diethyl-5-(pyrrolidin-1-yl)-1,2-dihydro-pyrazol-3-one,
4-Amino-5-(3-dimethylamino-pyrrolidin-1-yl)-1,2-diethyl-1,2-dihydro-pyrazol-3-one,
2,3-diamino-6-hydroxy-6,7-dihydro-1H,5H-pyrazolo[1,2-a]pyrazol-1-one.

A titre de bases hétérocycliques, on utilisera préférentiellement le 4,5-diamino 1-(β-hydroxyéthyl) pyrazole, la 2,3-diamino-6,7-dihydro-1H,5H-pyrazolo[1,2-a]pyrazol-1-one et leurs sels d'addition.

La ou les bases d'oxydation sont généralement présentes dans la composition colorante en quantité comprise entre 0,001 et 10 % en poids environ du poids total de la composition, de préférence entre 0,005 et 6 % en poids environ.

A titre d'exemples de coupleurs, on peut citer les méta-phénylènediamines, les méta-aminophénols, les méta-diphénols, les coupleurs naphtaléniques, les coupleurs hétérocycliques et leurs sels d'addition.

On peut notamment citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Le ou les coupleurs sont généralement présents dans la composition colorante en quantité comprise entre 0,001 et 10 % en poids environ du poids total de la composition, de préférence entre 0,005 et 6 % en poids environ.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telle que la soude, la potasse, l'ammoniaque, les amines ou les alcanolamines.

La composition oxydante selon l'invention peut également être utilisée dans un procédé de décoloration des fibres kératiniques, et en particulier des fibres kératiniques humaines telles que les cheveux.

Le procédé de décoloration selon l'invention comprend une étape d'application sur les fibres kératiniques d'une composition décolorante comprenant de préférence de l'eau oxygénée en milieu alcalin après mélange extemporané. Classiquement, une deuxième étape du procédé de décoloration selon l'invention est une étape de rinçage des fibres kératiniques.

La composition décolorante appliquée sur les fibres kératiniques peut être obtenue par mélange d'une composition oxydante selon l'invention avec une composition aqueuse ou anhydre contenant de préférence un ou plusieurs agents alcalins. La composition anhydre peut être pulvérulente ou sous forme de pate et dans les deux cas contient de préférence un ou plusieurs sels peroxygénés, et en particulier un ou plusieurs persulfates. La composition anhydre sous forme de pate contient de plus un ou plusieurs liquides inertes organiques.

Un autre objet de la présente invention est un procédé de déformation permanente des fibres kératiniques, et en particulier des fibres kératiniques humaines telles que les cheveux, utilisant une composition oxydante telle que définie ci-dessus.

Selon ce procédé, on applique sur les fibres kératiniques à traiter une composition réductrice, les fibres kératiniques étant mises sous tension mécanique avant, pendant ou après l'application de la composition réductrice, on rince éventuellement les fibres, on applique sur les fibres éventuellement rincées la composition oxydante de la présente invention puis on rince éventuellement à nouveau les fibres.

La première étape de ce procédé consiste à appliquer sur les cheveux une composition réductrice. Cette application se fait mèche par mèche ou globalement.

La composition réductrice comprend au moins un agent réducteur, qui peut être en particulier choisi parmi l'acide thioglycolique, la cystéine, la cystéamine, le thioglycolate de glycérol, l'acide thiolactique, ou les sels des acides thiolactique ou thioglycolique.

L'habituelle étape de mise sous tension des cheveux sous une forme correspondant à la forme finale désirée pour ces derniers (boucles par exemple) peut être mise en oeuvre par tout moyen, mécanique notamment, approprié et connu en soi pour maintenir sous tension des cheveux, tels que par exemple rouleaux, bigoudis, peignes et analogues.

Les cheveux peuvent également être mis en forme sans l'aide de moyens extérieurs, simplement avec les doigts.

Avant de procéder à l'étape suivante facultative de rinçage, il convient, de manière classique, de laisser reposer pendant quelques minutes, généralement entre 5 minutes et une heure, de préférence entre 10 et 30 minutes, la chevelure sur laquelle a été appliquée la composition réductrice, et ceci de façon à bien laisser le temps au réducteur d'agir correctement sur les cheveux. Cette phase d'attente est effectuée de préférence à une température allant de 35 °C à 45 °C, en protégeant de préférence également les cheveux par un bonnet.

Dans la deuxième étape facultative de rinçage, les cheveux imprégnés de la composition réductrice sont rincés soigneusement par une composition aqueuse.

Puis, dans une troisième étape, on applique sur les cheveux ainsi rincés la composition oxydante selon la présente invention, dans le but de fixer la nouvelle forme imposée aux cheveux.

Comme dans le cas de l'application de la composition réductrice, la chevelure sur laquelle a été appliquée la composition oxydante est ensuite, de manière classique, laissée dans une phase de repos ou d'attente qui dure quelques minutes, généralement entre 3 et 30 minutes, de préférence entre 5 et 15 minutes.

Si la tension des cheveux était maintenue par des moyens extérieurs, on peut retirer de la chevelure ces derniers (rouleaux, bigoudis et analogues) avant ou après l'étape de fixation.

Enfin, dans la dernière étape du procédé selon l'invention, étape facultative également, les cheveux imprégnés de la composition oxydante sont rincés soigneusement, généralement à l'eau.

La présente invention a également pour objet l'utilisation pour le traitement des fibres kératiniques, et en particulier des fibres kératiniques humaines telles que les cheveux, d'une composition oxydante telle que définie ci-dessus.

La présente invention a notamment pour objet l'utilisation pour la teinture des fibres kératiniques, et en particulier des fibres kératiniques humaines telles que les cheveux, d'une composition oxydante telle que définie ci-dessus.

La présente invention a également pour objet l'utilisation pour la décoloration des fibres kératiniques, et en particulier des fibres kératiniques humaines telles que les cheveux, d'une composition oxydante telle que définie ci-dessus.

La présente invention a aussi pour objet l'utilisation pour la déformation permanente des fibres kératiniques, et en particulier des fibres kératiniques humaines telles que les cheveux, d'une composition oxydante telle que définie ci-dessus.

Les exemples suivants illustrent l'invention sans présenter un caractère limitatif.

### EXEMPLES

Les compositions oxydantes suivantes se présentent sous la forme de crèmes lisses et homogènes dont l'évolution dans le temps est très faible, aussi bien au niveau de l'état de la dispersion (aspect microscopique, granulométrie) que des paramètres rhéologiques :

| Composition | 1 | 2 | 3 | 4 |
|---|---|---|---|---|
| Steareth-2 (Brij 72, Uniqema) | 4 g | - | 5 g | 5 g |
| Beheneth-5 (Nikkol BB-5, Nikko) | - | 4 g | - | - |
| Chlorure de béhényl-triméthylammonium à 79 % dans un mélange eau / isopropanol (Genamin KDMP, Clariant) | - | - | - | 1 g |
| Glycérol | - | - | 1,5 g | 1,5 g |
| Peroxyde d'hydrogène en solution aqueuse à 50 % | 12 g | 12 g | 4,8 g | 4,8 g |
| Salicylate de sodium | 0,035 g | 0,035 g | 0,035 g | 0,035 g |
| Acide étidronique, sel tétrasodique en solution aqueuse à 30 % | 0,2 g | 0,2 g | 0,2 g | 0,2 g |
| Pyrophosphate tétrasodique, 10 H₂O | 0,04 g | 0,04 g | 0,04 g | 0,04 g |
| Eau déminéralisée | q.s.p. | q.s.p. | q.s.p. | q.s.p. |
| | 100 g | 100 g | 100 g | 100 g |

Outre leur stabilité en conservation, leurs qualités d'usage sont également améliorées par rapport aux technologies existantes, notamment en termes d'opacité, d'aspect brillant et de comportement rhéologique.

### Exemple de coloration d'oxydation

Le mélange de la composition 1 ou de la composition 2 avec une crème de coloration d'oxydation (L'Oréal Majirouge ® nuance 6,64) en proportions pondérales 1 / 1,5 (crème colorante / composition oxydante) est particulièrement aisé à l'aide d'un pinceau et d'un bol et il conduit rapidement à une crème onctueuse et facile à appliquer sur les longueurs et les pointes d'une chevelure sèche et non lavée. Après un temps de pose de 35 minutes, un rinçage, un shampooing puis un séchage, l'éclaircissement est de deux tons et le résultat tinctorial est au moins équivalent à celui qui est obtenu avec une composition oxydante de l'art antérieur présentant la même force oxydante (correspondant à de l'eau oxygénée 20 volumes) et comprenant de l'eau, du peroxyde d'hydrogène, de l'alcool cétylstéarylique, de l'alcool cétylstéarylique oxyéthyléné à 30 moles d'oxyde d'éthylène et du monoéthanolamide d'acide alkyl C₁₃ / C₁₅ éther carboxylique à 2 moles d'oxyde d'éthylène mais ne comprenant pas de composé amphiphile non ionique à caractère hydrophobe utile dans le cadre de l'invention, soit la composition oxydant 1 20 volumes ® L'Oréal Professionnel (composition A). La chevelure présente un toucher particulièrement agréable.

### Exemple de permanente

Les cheveux sont lavés, essorés et enroulés sur des bigoudis. La composition réductrice Dulcia Tonica ® force Cheveux Naturels est appliquée sur l'ensemble de la chevelure et un temps de pause de 15 minutes est observé. La chevelure est rincée. La composition 3 est appliquée sur chaque bigoudi et un temps de pause de 5 minutes est observé. Les bigoudis sont enlevés et de la composition 3 est ajoutée sur l'ensemble de la chevelure en malaxant les pointes. Un temps de pause de 5 minutes est observé. La chevelure est rincée puis séchée. La chevelure présente des boucles régulières et un toucher très agréable.

### Exemple de lissage thiolé

Les cheveux sont lavés, essorés. La composition réductrice X Tenso ® force Cheveux Naturels est appliquée au pinceau ou à la main sur l'ensemble de la chevelure et un temps de pause de 30 minutes est observé. La chevelure est rincée. La composition 3 est appliquée au pinceau ou à la main sur l'ensemble de la chevelure et un temps de pause de 10 minutes est observé. La chevelure est rincée. La chevelure présente un aspect très lisse et un toucher très agréable.

Des résultats identiques ont été obtenus en substituant la composition 3 par la composition 4.

### Exemple de décoloration

Le mélange de la composition 1 ou de la composition 2 avec une poudre décolorante Platifiz ™ Precision est effectué en proportions pondérales 1 / 2,5 (poudre décolorante / composition oxydante) dans un bol à l'aide d'un pinceau, jusqu'à l'obtention d'une crème onctueuse. Cette crème est appliquée sur l'ensemble de la chevelure à l'aide du pinceau puis un temps de pose de 30 à 50 minutes est observé selon le niveau d'éclaircissement souhaité. La chevelure est ensuite rincée puis séchée. On obtient un niveau d'éclaircissement pouvant aller jusqu'à 7 tons, tout en conservant un toucher agréable.

### Exemple comparatif

La composition 1 a été comparée à la composition A décrite précédemment pour l'exemple de coloration d'oxydation. Après fabrication en cuve industrielle, les deux compositions se présentent sous forme de crèmes dont les viscosités peuvent être mesurées à 25,0 °C pour une vitesse de cisaillement de 70 s⁻¹ à l'aide d'un rhéomètre rotatif RS 1 (société ThermoFisher) équipé d'une géométrie en titane sablé de type cône-plan (diamètre 60 mm / angle 1 °). Après 25 minutes de cisaillement, les viscosités ainsi mesurées sont respectivement de 0,22 Pa.s pour la composition 1 et 0,17 Pa.s pour la composition A. Après deux mois de conservation à température ambiante, la composition 1 montre un aspect et une viscosité comparables à l'état initial (viscosité = 0,27 Pa.s) alors que la composition A présente un aspect pailleté et une viscosité bien inférieure à l'état initial (viscosité = 0,10 Pa.s). L'observation de la composition A au microscope optique en lumière blanche révèle la présence de cristallites massifs dont la taille est de l'ordre de 100 µm. Ces cristallites résultent de la recristallisation de l'alcool cétylstéarylique. Après deux mois de conservation à température ambiante, l'aspect microscopique de la composition 1 est identique à l'aspect initial.

## Revendications

1. Composition pour le traitement des fibres kératiniques sous forme de dispersion aqueuse comprenant, dans un milieu cosmétiquement acceptable :
- un ou plusieurs agents oxydants ;
- un ou plusieurs composés amphiphiles non ioniques à caractère hydrophobe solides à température ambiante ; et
- éventuellement un ou plusieurs agents tensioactifs à caractère hydrophile ;
le ou les agents tensioactifs à caractère hydrophile présents dans la composition étant cationiques ou zwittérioniques ;
le rapport pondéral entre les agents tensioactifs à caractère hydrophile et les composés amphiphiles non ioniques à caractère hydrophobe étant compris entre 0 et 0,16.

2. Composition selon la revendication 1 dans laquelle le ou les agents oxydants sont choisis parmi le peroxyde d'hydrogène, le peroxyde d'urée, les bromates alcalins, les polythionates et les persels.

3. Composition selon la revendication 1 ou 2 dans laquelle le ou les agents oxydants représentent de 0,1 à 50 % en poids par rapport au poids total de la composition oxydante.

4. Composition selon l'une quelconque des revendications 1 à 3 dans laquelle le ou les composés amphiphiles non ioniques à caractère hydrophobe comportent une ou plusieurs chaînes grasses saturées en C₁₂-C₃₀ reliées par une liaison éther, ester, amide ou carbamate à une tête polaire composée d'une ou plusieurs unités choisies parmi les unités oxyalkylène, sorbitane, sucre et autres polyols.

5. Composition selon l'une quelconque des revendications précédentes dans laquelle le ou les composés amphiphiles non ioniques à carractère hydrophobe ont une HLB calculée comprise entre 2 et 10.

6. Composition selon l'une quelconque des revendications précédentes dans laquelle le ou les composés amphiphiles non ioniques à carractère hydrophobe sont choisis parmi les composés suivants :
| Nom INCl | Référence commerciale | HLB calculée |
|---|---|---|
| STEARETH-2 | Brij 72 (Uniqema) | 4,9^{*} |
| STEARETH-3 | Isoxal 5 (Vevy) | 6,6^{*} |
| STEARETH-4 | Nikkol BS-4 (Nikko) | 7,9^{*} |
| STEARETH-5 | Jeecol SA-5 (Jeen) | 9,0^{*} |
| STEARETH-6 | Emalex 606 (Nihon Emulsion) | 9,9^{*} |
| CETETH-2 | Brij 52 (Uniqema) | 5,3^{*} |
| CETETH-3 | Emalex 103 (Nihon Emulsion) | 7,1^{*} |
| CETETH-4 | Lipocol C-4 (Lipo) | 8,4^{*} |
| CETETH-5 | Volpo C5 (Croda) | 9,5^{*} |
| CETEARETH-2 | Volpo CS2 (Croda) | 5,1^{*} |
| CETEARETH-3 | Jeecol CS-3 (Jeen) | 6,8^{*} |
| CETEARETH-4 | Lipocol SC-4 (Lipo) | 8,1^{*} |
| CETEARETH-5 | Volpo CS5 (Croda) | 9,2^{*} |
| BEHENETH-5 | Nikkol BB-5 (Nikko) | 8,1^{*} |
| COCAMIDE MEA | Comperlan 100 (Cognis) | 4,8 |
| COCAMIDE MIPA | Ninol M-10 (Stepan) | 5,6 |
| COCAMIDE DEA | Comperlan KD (Cognis) | 7,1 |
| STEARAMIDE MEA | Monamid S (Uniqema) | 3,7 |
| STEARAMIDE DEA | Lipamide S (Lipo) | 5,6 |
| MYRISTAMIDE DEA | Jeemide MRCA (Jeen) | 6,6 |
| MYRISTAMIDE MEA | Witcamide MM (Witco) | 4,4 |
| POLYGLYCERYL-2 DISTEARATE | Emalex DSG-2 (Ikeda) | 4,7 |
| POLYGLYCERYL-3 DISTEARATE | Cithrol 2623 (Croda) | 6,2 |
| POLYGLYCERYL-2 STEARATE | Nikkol DGMS (Nikko) | 7,6 |
| POLYGLYCERYL-3 STEARATE | Radiasurf 7248 (Atofina) | 9,4 |
| PEG-2 STEARATE | Sedefos 75 (Gattefosse) | 5,6 |
| PEG-3 STEARATE | Tegin D 1102 (Goldschmidt) | 7,2 |
| PEG-4 STEARATE | Cithrol 2MS (Croda) | 8,4 |
| SORBITAN DISTEARATE | Sorbon S-66 (Toho) | 4,7 |
| SORBITAN PALMITATE | Span 40 (Uniqema) | 8,1 |
| SORBITAN STEARATE | Span 60 (Uniqema) | 7,6 |
| SORBITAN TRISTEARATE | Span 65 (Uniqema) | 3,3 |
| MYRISTYL GLUCOSIDE | Montanov 14 (Seppic) | 9,5 |
| CETEARYL GLUCOSIDE | Tego Care CG 90 (Degussa) | 8,6 |
| ARACHIDYL GLUCOSIDE | Montanov 202 (Seppic) | 7,8 |
| | | |
|---|---|---|
| ^{*} correspondant à la HLB calculée selon la méthode de Griffin | | |

7. Composition selon l'une quelconque des revendications précédentes dans laquelle le ou les composés amphiphiles non ioniques à caractère hydrophobe représentent de 2 à 30 % en poids par rapport au poids total de la composition oxydante.

8. Composition selon l'une quelconque des revendications précédentes dans laquelle le ou les agents tensioactifs à caractère hydrophile ont une HLB calculée comprise entre 10 et 50.

9. Composition selon l'une quelconque des revendications précédentes ne comprend pas d'agent tensioactif à caractère hydrophile.

10. Composition selon l'une quelconque des revendications précédentes comprenant un ou plusieurs alcools gras en C₁₂-C₃₀ de HLB calculée inférieure à 2.

11. Composition selon la revendication 10 dans laquelle le ou les alcools gras sont choisis parmi les alcools non (poly)oxyalkylénés et non (poly)glycérolés, comprenant une ou plusieurs chaînes grasses présentant de 12 à 30 atomes de carbone, linéaires ou ramifiés, saturés ou insaturés, les chaînes grasses étant éventuellement substituées par un ou deux groupements hydroxyle supplémentaires.

12. Composition selon la revendication 10 ou 11 dans laquelle le ou les alcools gras en C₁₂-C₃₀ représentent de 0 à 10 % en poids par rapport au poids total de la composition oxydante.

13. Composition selon la revendication 12 ne comprenant pas d'alcool gras en C₁₂-C₃₀.

14. Procédé de traitement des fibres kératiniques comprenant l'application sur les fibres kératiniques d'une composition oxydante telle que définie à l'une quelconque des revendications 1 à 13.

15. Utilisation pour le traitement des fibres kératiniques d'une composition oxydante telle que définie à l'une quelconque des revendications 1 à 13.
